# EUROPEAN PATENT SPECIFICATION

(11) **EP 0 339 040 B1**
(45) Date of publication and mention of the grant of the patent: **14.09.1994**
(21) Application number: 88900562.5
(22) Date of filing: 23.12.1987
(51) Int. Cl.: A61K 39/02

(54) **SWINE DYSENTERY VACCINE**
IMPFSTOFF GEGEN SCHWEINEDYSENTERIE
VACCIN CONTRE LA DYSENTERIE PORCINE

(30) Priority: 23.12.1986 AU 9632/86; 24.08.1987 AU 3940/87
(43) Date of publication of application: 02.11.1989
(73) Proprietor: ROYAL MELBOURNE INSTITUTE OF TECHNOLOGY LIMITED, Melbourne, VIC 3000 (AU)
(72) Inventor: COLOE, Peter, John, East Doncaster, VIC 3109 (AU)
(74) Representative: Holmes, Michael John
(86) International application number: AU8700440
(87) International publication number: WO8804555

(56) References cited:
- EP-A- 155 790
- JP-A- 6 214 797
- US-A- 4 100 272
- US-A- 4 152 415
- US-A- 4 203 968
- US-A- 4 469 672
- BIOLOGICAL ABSTRACTS, vol. 11, 1975; M.J. HUDSON et al., acc. no. 11000663#
- BIOLOGICAL ABSTRACTS, vol. 84, no. 3, 1987, Biological Abstracts Inc., Philadelphia, PA (US); E.M. JENKINS et al., acc. no. 28415#
- RESEARCH IN VETERINARY SCIENCE, vol. 21, 1976; M.J. HUDSON et al., pp. 366-367#
- Can. J. Biochem and Physiology, Vol. 40, Page 1129, (1962), Hagen, J.H. and Hagen, P.B..
- Maruo Fumiharu, Tamiya Nobuo, Asakura Kunizo Kanshu (Koso Handbook)1 December 1982 (01.12.82) Asakura Shoten p. 134-135, 259

## Description

This invention relates to the immunisation of pigs against swine dysentery, and in particular it relates to a vaccine for the control of this disease.

An anaerobic spirocheate, Treponema hyodysenteriae, has been isolated and identified as the primary aetiological agent of swine dysentery, a mucoid, haemorrhagic diarrhoea of swine that is worldwide in distribution. The organism has a predeliction for the large intestine (colon) of swine where it proliferates, in the presence of other anaerobic bacteria, and produces a mucoid, haemorrhagic to necrotic degeneration of large portions of the large intestine. There is extensive invasion of the epithelia and lamnia propria of the tissue and the spirochaetes are clearly obvious, under light or electron microscopy, aligned along the necrotic tissue.

There is virtually no information available on the immune response of pigs to T.hyodysenteriae infection although it has been demonstrated that pigs that have recovered from swine dysentery are refractory to further challenge.

Several attempts have been made to develop an effective swine dysentery vaccine. U.S. Patent 4,100,272 describes a vaccine comprising chemically killed cells of a virulent (pathogenic) isolate of Treponema hyodysenteriae for parenteral administration, whilst U.S. Patent 4,152,413 describes a similar vaccine comprising killed cells of a virulent isolate for oral administration. U.S. Patent Nos. 4,152,415 and 4,469,672 describe modification of the oral vaccination procedure which include the step of parenterally administering the vaccine prior to the oral administration thereof.

International Publication no. W085/03875 discloses a modified vaccination regime which comprises parenteral administration of a priming dose of a killed vaccine, and at about the same time or thereafter the oral administration of a live, avirulent or non-pathogenic strain of T.hyodysenteriae.

It will be apparent that in these prior vaccination attempts, killed virulent strains of T.hyodysenteriae have been used and the cells have been treated with fixative chemicals such as formaldehyde. In all instances, the killed vaccine has been administered either alone by injection or orally, or by injection in conjunction with oral administration of a killed virulent strain or a live avirulent strain. The rationale of the oral administration of the avirulent strain is to stimulate the local immunity of the large intestine, and presumably to stimulate an IgA response.

Hudson et al., in Research in Veterinary Science, 1976, 21: 366-367, describe a combined vaccination regime combining oral or intra-peritoneal administration of live attenuated. T.hyodysenteriae together with intramuscular injection of a formalin-killed strain.

It has now been found that effective immunisation of pigs against T.hyodysenteriae infection can be achieved by intra-muscular administration of oxygen-treated, non-viable strains of T.hyodysenteriae. Furthermore, the strains which may be used in accordance with the present invention include both virulent and attenuated strains.

Accordingly, the present invention provides a parenteral vaccine composition for the immunisation of pigs against Treponema hyodysenteriae infection, preferably by intramuscular, administration, which comprises an oxygen-treated, non-viable strain of T.hyodysenteriae, optionally with an adjuvant, in an acceptable carrier therefor adapted for parenteral administration. As previously mentioned, either a virulent or attenuated strain of T.hyodysenteriae may be used in this method, and as described below, representative strains which may be used in accordance with the present invention include reference virulent strains deposited at the American Type Culture Collection under ATCC Nos. 31287 and 31212, and the reference avirulent strain deposited under ATCC No. 27164 (see U.S. Patent Specification No. 4,100,272).

The strains which can be used for parenteral, particularly intramuscular, administration in accordance with the present invention include any isolate of T.hyodysenteriae with a protein profile similar to the profiles of virulent strain 5380 or attenuated strain 70A including reference strains ATCC 31287, 31212 and 27164 as previously described. As described in greater detail below, minor differences in the protein profile do not appear to have a major influence on the immunogenicity of various virulent or attenuated strains, and the use of virulent strain 5380 and attenuated 70A has been described in detail herein as representative of the isolates of virulent and attenuated strains which can be used in accordance with the present invention. Outer membrane protein profiles show marked similarity of all T.hyodysenteriae isolates and Western blot analysis of immunogenic proteins show that antisera raised to the virulent T.hyodysenteriae isolate 5380 recognise immunogenic proteins in the same molecular weight region in all T.hyodysenteriae isolates.

No detectable differences can be observed in the protein profile of oxygenated T.hyodysenteriae compared to live, non-oxygenated T.hyodysenteriae. Similarly western blot analysis of immunogenic proteins show that antisera raised to virulent T.hyodysenteriae isolate 5380 recognise the same immunogenic proteins irrespective of whether the cells have been oxygen treated or are live. The fact that the oxygen treatment results in no change in the protein profile, and hence no change in antigenicity appears to be significant, as in prior chemically killed vaccines (such as formalin-killed vaccines) it is believed that the chemical treatment destroys certain antigens including some surface antigens of T.hyodysenteriae.

The T.hyodysenteriae isolates used for preparation of the vaccine of this invention can be grown using trypticase soy broth incubated at 37-38°C under an anaerobic atmosphere such as 50% H₂/tp% CO₂, or 10% CO₂ in N₂. Cysteine or other reducing compounds are added to the medium to maintain anaerobiosis and, when cultures are first being grown in liquid medium, it may be necessary to supplement the medium with fetal calf serum (up to 10%), glucose, citrate, pyruvate, and iron (at 2µg/ml of medium, crucial concentration). The liquid medium may be supplemented with spectinomycin at 400µg/ml to suppress possible contamination without changing the protein composition of the isolated cells. If desired, the cells may then be rendered non-viable by treatment with oxygen as described below.

Alternatively, T.hyodysenteriae cells may be grown on blood agar plates under anaerobic conditions. When the cells are prepared in this manner, they must be washed in phosphated buffered saline or other isotonic solutions to remove impurities in the agar before use and then, if desired, held in oxygen saturated solution to render them non-viable.

The cultures of T.hyodysenteriae can be grown in the temperature range 35°C to 42°C and there is no difference in the protein composition of strains grown over this temperature range.

After T.hyodysenteriae cells have been grown in a fermenter they can be harvested by centrifugation and suspended in phosphate buffered saline (0.1M) or normal saline (0.1M) pH 7.0-7.4 and stored at either -70°C or -20°C in the concentrated form. Alternatively the cells can be aliquoted into smaller volumes and freeze dried.

To kill T.hyodysenteriae cells that have been grown in a fermentor, they are preferably oxygenated by bubbling 0₂ gas through the medium to saturate the medium with 0₂. The oxygenation procedure is preferably maintained for at least 3-4 hours, more preferably up to 6 hours, to render all T.hyodysenteriae in the medium non-viable. The cells can be used directly after oxygenation by combining with adjuvant or alternatively they can be harvested by centrifugation, and suspended, and stored in the concentrated form as previously described. Alternatively the cells can be aliquoted into smaller volumes and freeze dried. The cell density for vaccination should be approximately 10⁹ organisms/ml. The vaccine preferably comprises 1ml of 1 x 10⁹ organisms mixed with 1ml of a suitable adjuvant, such as Freunds incomplete adjuvant (CSL). The vaccine may also include a preservative such as thimerosal. It is recommended that the vaccine be administered by deep intramuscular injection as this procedure provides the greatest immunological response, however, other accepted methods for parenteral administration of vaccines such as subcutaneous injection may be employed, but are not preferred.

To increase the resistance of swine to T.hyodysenteriae infection it is preferable to deliver the vaccine as two (2) doses of approximately 1 x 10⁹ T.hyodysenteriae administered 7-14 days apart. If the degree of immune response does not reach a level of greater than 1.0 OD Unit at a 1/800 dilution of serum as determined by an ELISA assay or equivalent serological assay after the second dose, a third dose of vaccine may be administered.

It is desirable in breeding stock to revaccinate animals with a yearly booster dose to maintain their IgG level above 1.00 OD Unit at a 1/800 dilution of serum.

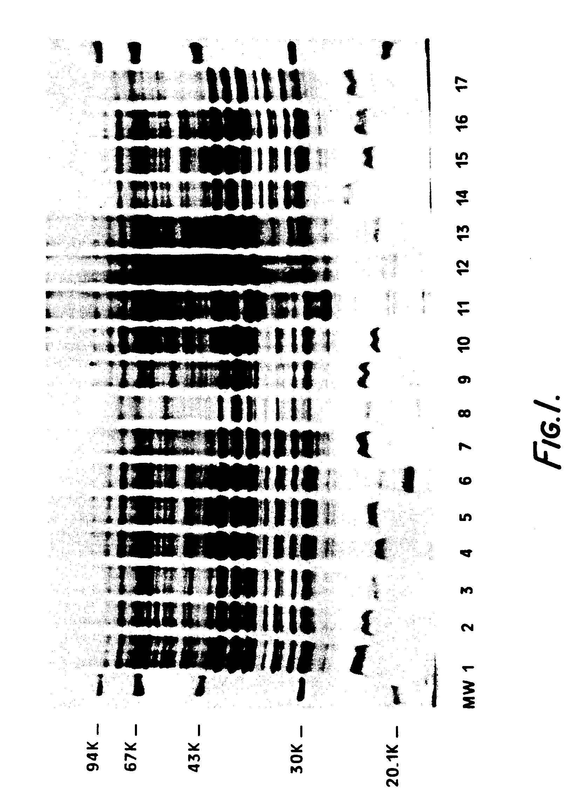

Accompanying Figures 1 to 5 depict the results of an examination of the SDS-PAGE profiles of whole cell and outer-membrane (OM) enriched fractions of a number of clinical isolates of T.hyodysenteriae and reference strains ATCC 31287, 31212 and 27164.

In the Figures:
Figure 1 shows SDS-PAGE profiles of whole cell lysates of isolates and reference strains of T.hyodysenteriae. Lanes 1 and 17, isolate strain, 70A; lane 2, isolate strain 5380; lane 3, isolate strain 5541; lane 4, isolate strain 32386; lane 5, isolate strain 32486A; lane 6, isolate strain 32486B; lane 7, reference strain ATCC 31212; lane 8, reference strain ATCC 27164; lane 9, reference strain ATCC 31287; lane 10, isolate strain 1545; lane 11, isolate strain 9690; lane 12, isolate 8841; lane 13, 8441; lane 14, isolate strain 508; lane 15, isolate strain 1059; lane 16, isolate strain 2549.

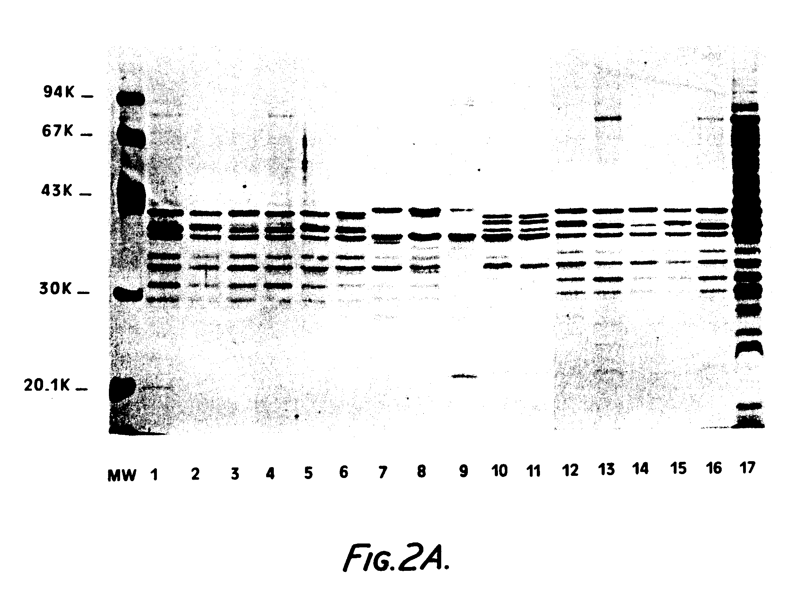

Figure 2 shows SDS-PAGE profiles of Sarkosyl-insoluble OM preparations. Panel A, 10 microgram of OM proteins from T.hyodysenteriae isolates. Lane 1, 70A; lane 2, ATCC 31287; Lane 3, ATCC 27164; lane 4, 2549; lane 5, 1545; lane 6, 5380; lane 7, 9690; lane 8, 8841; lane 9, 8441; lane 10, T.innocens 9509; lane 11, T.innocens 9510; lane 12, 1039; lane 13, ATCC 31212; lane 14, 32486B; lane 15, 508; lane 16, repeat 70A; lane 17, 5380 whole cell lysate. Panel B, 10 microgram of OM proteins from T.hyodysenteriae, E.coli, S.Typhimurium, Y.enterocolitica, C.jejuni and C.fetus. Lanes 1, 2 and 3 T.hyodysenteriae isolates 70A, ATCC 31287, 5380, respectively; lane 4, E.coli JP777, lane 5; S.typhimurium V279; lane 6, Y.enterocolitica 430-1; lane 7, C.jejuni F1; lane 8, C.jejuni F14, lane 9; C.fetus subsp.fetus; lane 10, repeat 5380.

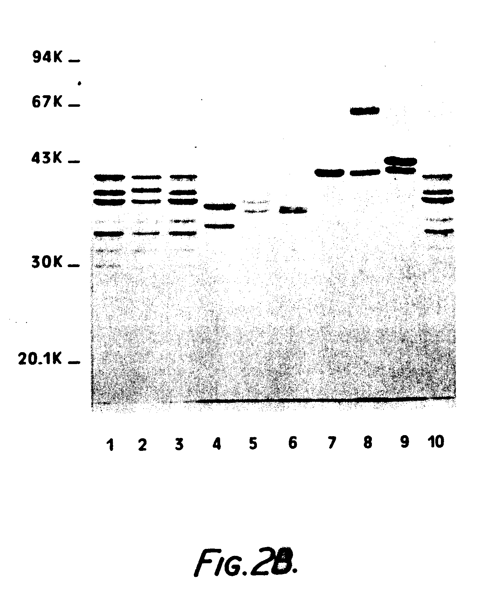

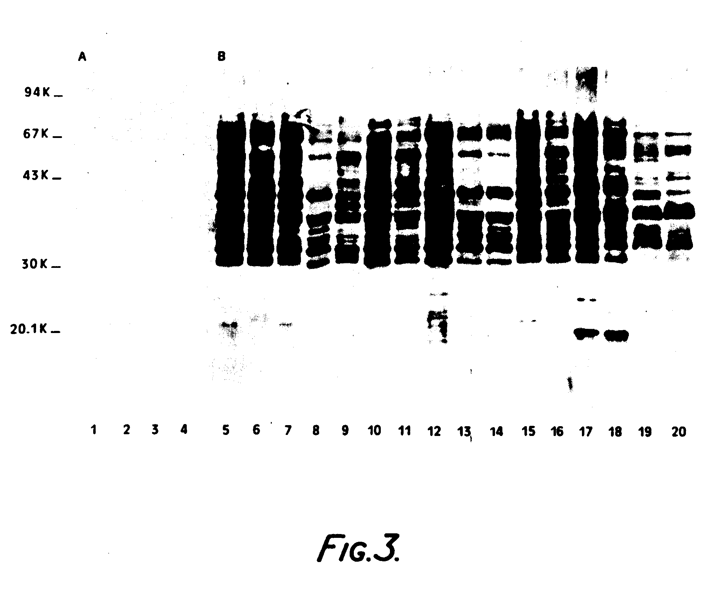

Figure 3 shows Western immunoblot analyses of T.hyodysenteriae whole cell solubilised proteins using specific porcine serum. Cell lysates were separated by SDS-PAGE and transferred to zetaprobe for Western blot analyses. Antigens were detected using pre-immune control serum (Panel A) and polyvalent porcine anti-T.hyodysenteriae (isolate 5380) serum (Panel B) diluted 1:100. Lanes 1 and 5, 70A; lanes 2 and 6, 5380; lanes 3 and 7 ATCC, 31287; lanes 4 and 8, 32386; lane 9, 1545; lane 10, ATCC 31212; lane 11, 1059; lane 12, repeat 5380; lane 13, 5541; lane 14, 32486B; lane 15, repeat ATCC 31287; lane 16, ATCC 27164; lane 17, 9690; lane 18, 8841; lane 19, T.innocens 9510; lane 20, T.innocens 9509. Bound antibody was detected using (³⁵S) Protein a and subsequent autoradiography.

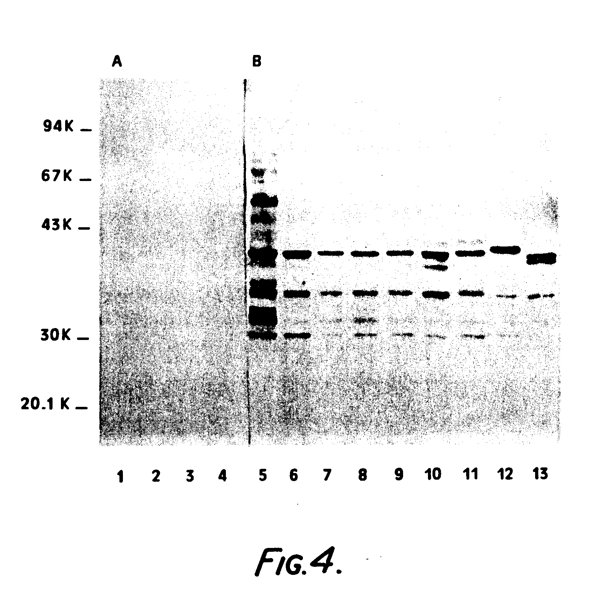

Figure 4 shows Western blot profiles of OM proteins of T.hyodysenteriae isolates using porcine anti-T.hyodysenteriae serum. OM proteins were prepared by Sarkosyl solubilisation. Panel A, Immune blot with pre-immune control sera; Panel B, Immune blot with hyper-immune sera. Serum was diluted 1:100. Lanes 1 and 5, T.hyodysenteriae 5380 cell extract; lanes 2 and 6, 5380 OMP; lanes 3 and 7, ATCC 31287 OMP; lanes 4 and 8, 70A OMP; lane 9, 2549 OMP; lane 10, 1545 OMP; lane 11, ATCC 27164 OMP; lane 12, 8841 OMP; lane 13, T.innocens 9510 OMP. Antibody binding was detected by incubation with (³⁵S)-Protein A and subsequent autoradiography.

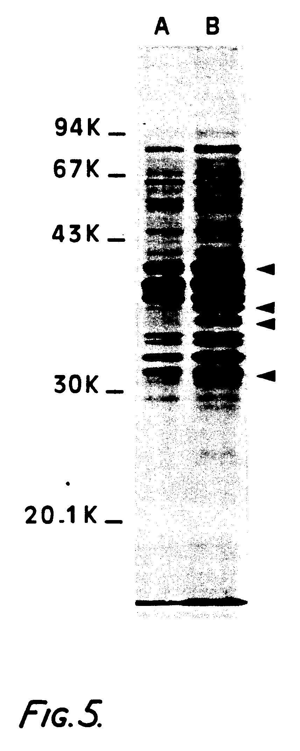

Figure 5 shows the effect of protease digestion on T.hyodysenteriae isolate 5380 whole cells. Intact cells were incubated with 50 µg/ml of trypsin (lane A) or with PBS buffer (lane B). After 30 min, reactions were stopped by the addition of PMSF and cells solubilised in SDS sample buffer prior to SDS-PAGE. Arrows indicate the position of the Coomassie blue-stained proteins sensitive to proteolysis.

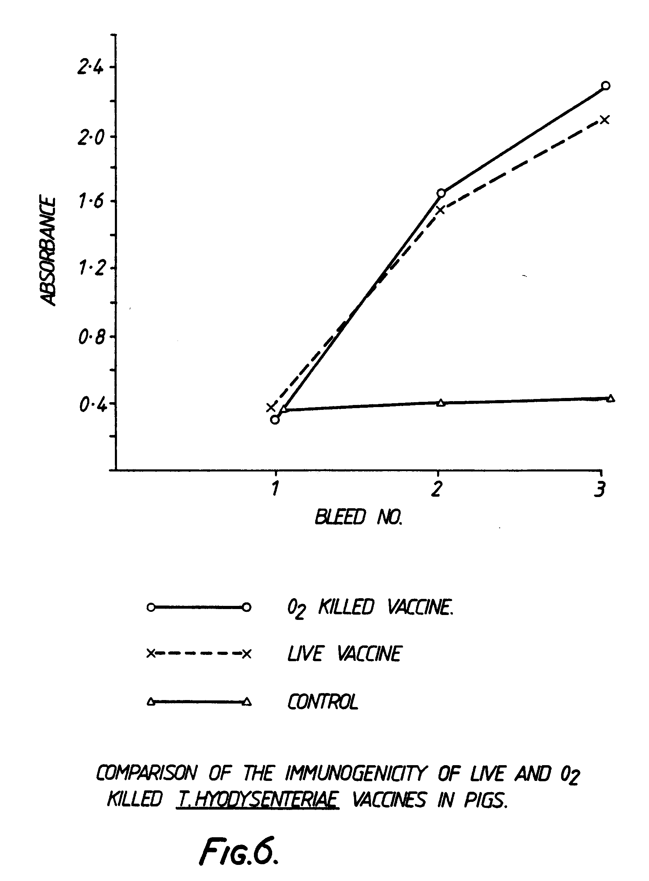

Figure 6 shows a comparison of the immunogenicity of live and oxygen-killed T.hyodysenteriae vaccines in pigs. (Abscissa shows bleed No.; ordinate shows absorbance; 0-0 O₂ killed vaccine; X---X live vaccine; △-△ control.)

### RESULTS

SDS-PAGE profiles of polypeptides of whole-cell lysates from clinical isolates and type strains of T.hyodysenteriae showed four to seven major abundant proteins in the molecular mass (Mr) range of 30kDa to 40kDa and at least 30 additional less intense Commassie blue stained proteins were resolved for each strain on SDS-PAGE. No high M.Wt. polypeptides (> 200 kDa) were observed in the electrophoretic profiles. The 36kDa and 39kDa proteins were detected as closely migrating doublets in most isolates. In addition a diffuse band present in all strains in the M.Wt. region 22-25kDa was identified as lipo-polysaccharide (LPS) on the basis of the silver staining and nitrocellulose binding characteristics (data not shown).

Comparison of the Coomassie blue stained protein profiles of T.hyodysenteriae whole-cell lysates demonstrated qualitative differences in the apparent electrophoretic mobility of the major abundant proteins migrating in the M.Wt. region of the SDS-PAGE gel between 30kDa to 40kDa. This strain-to-strain variability in the banding patterns could be used to differentiate two electrophoretic patterns among the isolates tested. The majority of treponemal strains examined revealed similar protein banding profiles to that of type strains ATCC 31212 and ATCC 31287 (Fig.1, lanes 7 and 9, respectively); and were designated group A, with characteristic major abundant proteins with molecular masses of 39kDa, 37KDa and 36kDa proteins. Type strain ATCC 27164 (Fig.1, lane 8) illustrated an identical polypeptide profile to strain ATCC 31287 but did not express the 39kDa protein doublet (also designated group A). A distinct 40kDa protein was expressed in isolates 9690, 8841 and 8441 (Fig.1, lanes 11, 12 and 13, respectively) and these isolates were designated group B.

Four different preparations of whole-cell lysates from the same T.hyodysenteriae strains (950A, 70A, 31287, 31212) were examined by SDS-PAGE after numerous passages on growth medium. All strains showed identical polypeptide profiles demonstrating that the protein composition of the isolates did not vary on passage (data not shown). Furthermore the protein profile of strain 5380 (group A (Fig.1, lane 2) remained stable after in vivo challenge and subsequent re-isolation from several pigs (data not shown).

Cell envelope fractions from the clinical isolates and type strains of T.hyodysenteriae and the two isolates of T.innocens were extracted with Sarkosyl and Sarkosyl-insoluble, OMP-enriched preparatins resolved by SDS-PAGE (Fig.2A). The major OM proteins present in T.hyodysenteriae strains corresponded to the major abundant proteins migrating in the Mr range 30kDa to 40kDa in the cellular protein preparations (Refer to fig.1). The OMP profiles revealed that all group A and most group B isolates expressed common 36kDa, 34kDa, 33kDa, 31.5kDa and 30kDa OM proteins. Of 10 group A strains tested, 6 expressed a major 37kDa OMP and 4 expressed a major 37.5kDa OMP. These isolates have been designated sub-groups A1 and A2, respectively. There was no expression of the strain variable 37kDa protein in Group B OMPs. The OMP proteins of the T.hyodysenteriae isolates were compared with the Sarkosyl-insoluble OMP-enriched preparations from other gram-negative bacteria (Fig.2B). The OM patterns of T.hyodysenteriae isolates were readily distinguished from those of E.coli, S.typhimurium, Y.enterocolitica, C.jejuni and C.fetus subsp.fetus.

Samples of treponemal whole-cell lysates were separated by SDS-PAGE and then transferred to zetaprobe for immuno-detection. Porcine hyperimmune serum against whole T.hyodysenteriae strain 5380 cells (Group A) recognised at least 20 proteins with apparent molecular masses above 24kDa, with some strain variability (Fig.3). No proteins were recognised by the pre-immune control serum. The hyper-immune porcine serum reacted strongly with a number of proteins in the molecular mass range 30kDa to 47kDa that were common in almost all isolates (Groups A and B) but different to the immunoreactive proteins recognised in the T.innocens isolates.

When Sarkosyl-insoluble OM preparations from seven T.hyodysenteriae (6 group A and 1 group B) isolates and a T.innocens isolate were separated by SDS-PAGE and immunoblotted to zetaprobe and probed with porcine anti-T.hyodysenteriae strain 5380 hyperimmune serum, the pattern of reactivity was similar in almost all isolates except for a small degree of strain-variatin in the proteins in the 38kDa to 40kDa dalton molecular mass range (Fig.4). Included for comparative purposes was the pattern of reactivity with isolate 5380 whole-cell lysate (Fig.4), lanes 1 and 5). The OM proteins from groups A and B and the T.innocens isolates were reactive with antibodies present in the hyperimmune sera but not with prechallenge control serum (Fig.4), lanes 1 to 4). In all T.hyodysenteriae isolates, strongly immunoreactive common OM proteins were observed at 34kDa and 30kDa. The 34kDa band was also reactive in the T.innocens isolate (Fig.4, lane 13). The 39kDa band present in group A isolates (Fig.4, lanes 6 to 11), the major 40kDa in group B isolate 8841 (Fig.4, lane 12) and the 38KDa and 38.5kDa proteins bands in the T.innocens isolate 9510 (Fig.4 lane 13) were also immunoreactive. A similar antibody response was observed when OM proteins were probed with rabbit anti-T.hyodysenteriae hyperimmune serum (data not shown). When OM proteins from other gram-negative organisms were immunoprobed with the same porcine anti-T.hyodysenteriae hyperimmune serum there was no detectable antibody cross-reactivity (data not shown).

To further characterise the T.hyodysenteriae antigens important in the immunological response, the OM proteins recognised in the Western blots were correlated with proteins located on the surface of the T.hyodysenteriae. Intact T.hyodysenteriae strain 5380 whole cells were treated with trypsin and the proteolysis stopped with PMSF. Both protease-treated and control T.hyodysenteriae 5380 cells were solubilised in SDS sample buffer and subjected to SDS-PAGE (Fig. 5). Trypsin proteolysis caused selective loss of the protein bands at Mr values of 39kDa, 36kDa, 34kDa (doublet), and 30kDa indicating that these proteins extend out through the OM to the treponemal cell surface. The same pattern of proteolysis was observed when proteinase K treatment of the T.hyodysenteriae 5380 cells was used rather than trypsin (data not shown).

Further features of the present invention are illustrated by the following experimental example.

### COMPARATIVE EXAMPLE 1

A. Experimental Animals. Pigs from a property with no history of swine dysentery were placed in isolation units at 4 weeks of age and fed a grower ration containing no antibiotics. Rectal swabs were examined by dark field microscopy and inoculated onto bovine and horse blood agar with and without spectinomycin and into selenite broths. No T.hyodysenteriae like organisms were observed under microscopy or isolated on the isolation media. Faeces were negative for Salmonella spp and Compylobacter spp. All animals were negative in a T.hyodysenteriae ELISA assay disclosed in the copending Australian Patent Application No. PH 09631/86, now issued as Australian Patent No. 11025/88.
B. Vaccine preparation. Cultures of T.hyodysenteriae, strains 5380 or 70A were grown for 48 hours in an anaerobically prepared Trypticase soy broth medium supplemented with cysteine, glucose, sodium citrate, sodium pyruvate, and iron, adjusted to pH 6.8, sterilized and gassed with a mixture of 10% CO₂ in N₂. Up to 10%, preferably 1%, fetal calf serum may be added to enhance growth. Cultures were harvested, and resuspended in phosphate buffered saline. The organisms were diluted to a concentration of approximately 1 x 10⁹/ml before use.
C. Challenge Inoculum. Challenge inoculum consisted of the same T.hyodysenteriae organisms grown in liquid culture, (as above) or a single blood agar plate of the organism which had been incubated for 48 hours at 37°C.
The challenge inoculum was fed in a homogenate of 200ml of reconstituted milk powder to pigs which had been deprived access to food for 24 hours prior to inoculation.
D. Virulence Trials
Trial 1.
Four pigs were incolulated orally with the growth from 1 blood agar culture of T.hyodysenteriae strain 5380. The pigs were maintained on an antibiotic free diet and were monitored daily for signs and symptoms of dysentery.
The clinical response of these pigs inoculated with T.hyodysenteriae 5380 was as follows:

| | |
|---|---|
| Diarrhoea | 4/4 |
| Dysentery | 4/4 |
| Av.Day of onset | 10 days |
| Av.Duration | 3 days |
| Deaths | 4/4 |

This trial showed that strain 5380 is virulent for pigs, and T.hyodysenteriae was isolated from all pigs with dysentery.
Trials 2 and 3
These were repeats of Trial 1 using six 3-week-old pigs. All pigs showed diarrhoea and dysentery 11 days post inoculation. In trial 2, two pigs died from dysentery on day 12 and the remainder, with classical signs of swine dysentery were treated with antibiotics to control the dysentery. Trial 3 results were similar.
Again these trials illustrated that strain 5380 is a virulent strain of T.hyodysenteriae
Trial 4
Eight pigs were challenged with cultures of 70A rather than 5380 using the same inoculation protocol as an trials 1, 2 and 3. No pigs succumbed to swine dysentery.
Trial 5
Six 4-week-old pigs were inoculated with either strain 5380 (4 pigs) or strain 70A (2 pigs) by oral inoculation of a concentrated inoculum (1 x 10¹¹) of the T.hyodysenteriae strains orally and the freshly harvested surface growth from 1 agar plate intrarectecally. The pigs were housed in separate crates in the same shed and fed the same ration. All 4 pigs from the 5380 group showed evidence of clinical swine dysentery within 10 days of challenge and 1/4 died at 12 days. The remainder of the 5380 group were treated with antibiotics to control the dysentery. Neither of the pigs challenged with strain 70A showed any signs of dysentery up to 30 days post challenge, when the experiment was terminated.
Trial 6
Eight pigs were challenged with cultures of 70A by inoculation of 1 agar plate of actively growing T.hyodysenteriae 70A in milk orally and the surface growth of actively growing T.hyodysenteriae 70A, resuspended in 1ml of T.hyodysenteriae culture broth and inoculated intrarectally. None of the pigs showed any signs of swine dysentery. However, they did show an increase in antibody titre against T.hyodysenteriae. T.hyodysenteriae could be isolated from the pig faeces in the 24 hours post inoculation. However, T.hyodysenteriae could not be isolated from faeces from 5 days after challenge.
E. Vaccination Trials
Trial 7
Six pigs were vaccinated intramuscularly with 2 x 10⁹ T.hyodysenteriae 5380 in adjuvant at day 0 and revaccinated on day 11. A control group of 6 pigs, kept separately, were untreated.
All pigs were challenged at day 22 with strain 5380 challenge fed in milk as per Trial 1. All control pigs died of swine dysentery within 17 days suffering from classical swine dysentery. All vaccinated pigs were resistant to challenge and showed no evidence of T.hyodysenteriae infection.
Trial 8
Five pigs were vaccinated as in Trial 7 but received the second vaccination only 10 days after the initial vaccination and were then challenged on day 26 (16 days after the second vaccination) with T.hyodysenteriae 5380. Unvaccinated pigs began to show signs of swine dysentery within 9 days of challenge and pigs started to die of acute swine dysentery within 10 days of challenge. None of the vaccinated pigs showed any evidence of swine dysentery infection up to 45 days post challenge. T.hyodysenteriae was isolated from all infected pigs which died but was not isolated from vaccinated pigs 12 days after challenge.
The clinical response of pigs in Trial 8 was as follows:

| | Unvaccinated | Vaccinated |
|---|---|---|
| Diarrhoea | 5/5 | 0/6 |
| Dysentery | 5/5 | 0/6 |
| Death | 5/5 | 1/6 * |

| | | |
|---|---|---|
| (* 1 pig died of E.coli peritonitis. There was no evidence of T.hyodysenteriae infection in the intestine and no T.hyodysenteriae was isolated from the intestinal tissue.) | | |

Blood was collected from all pigs in the trial and the serological titres (IgG levels) are shown in the accompanying figure (Fig.4).
Trial 9
Because polyacrylamide gel electrophoretic analysis of the soluble cellular proteins and outer membrane proteins of T.hyodysenteriae isolates 5380 and 70A did not reveal any significant differences in their protein composition or antigenicity as determined by a Western blot analysis (see Figures 1, 2 and 3) seven pigs were vaccinated with 2 x 10⁹ T.hyodysenteriae 70A at day 0 and revaccinated with 2 x 10⁹ T.hyodysenteriae 70A on day 17. Six pigs were kept separately as controls.
All pigs were challenged on day 24 (9 days after the second vaccination) with T.hyodysenteriae 5380. All control pigs showed signs of diarrhoea and dysentery, with all pigs dying of acute swine dysentery within 28 days after challenge.
None of the vaccinated pigs showed any signs of swine dysentery. One vaccinated pig did die during this trial on day 36, 12 days after challenge, with acute peritonitis. Examination of the intestinal tract did not show any evidence of swine dysentery and no T.hyodysenteriae was isolated from the large intestine. Microscopic, histological and electron microscopic analysis of unvaccinated pigs revealed changes in all pigs characteristic of acute swine dysentery - similar analysis of vaccinated pigs at the completion of the trial showed no evidence of microscopic or histological changes caused by T.hyodysenteriae infection.

Serological responses of vaccinated and unvaccinated pigs revealed marked differences in the IgG titres to T.hyodysenteriae in infected and uninfected pigs In all vaccinated pigs the serological titre (IgG) to T.hyodysenteriae was greater than 1.0 OD unit at a 1/800 dilution of serum. These vaccination trials demonstrate in pigs vaccinated with the virulent (5380) or avirulent (70A) strains of T.hyodysenteriae that an antibody titre (in excess of 1.0 at 1/800) can be obtained that will protect against challenge with a virulent strain (5380) of T.hyodysenteriae.

### EXAMPLE 1

A. Experimental Animals. Pigs from a property with no history of swine dysentery were placed in isolation units at 4 weeks of age and fed a grower ration containing no antibiotics. Rectal swabs were examined by dark field microscopy and inoculated onto bovine and horse blood agar with and without spectinomycin and into selenite broths. No T.hyodysenteriae like organisms were observed under microscopy or isolated on the isolation media. Faeces were negative for Salmonella spp and Compylobacter spp. All animals were negative in a T.hyodysenteriae ELISA assay as described in copending Australian Patent Application No. PH09631/86
B. Oxygen Treated Vaccine preparation Cultures of T.hyodysenteriae, strain 70A were grown for 48 hours in an anaerobically prepared Trypticase soy broth medium supplemented with cysteine, glucose, sodium citrate, sodium pyruvate, and iron, adjusted to pH 6.8, sterilized and gassed with a mixture of 10% CO₂ in N₂. Up to 10%, preferably 1%, fetal calf serum may be added to enhance growth.
Cultures were oxygenated for six hours, diluted to a concentration of approximately 1 x 10⁹/ml, and adjuvanted before use.
C. Vaccination Trial
One hundred and ten pigs were vaccinated with oxygen-treated, non-viable, adjuvanted T.hyodysenteriae vaccine at day 0 and day 10, and 110 pigs were kept as control pigs. The pigs were all housed in a commercial piggery with endemic swine dysentery.
Unvaccinated pigs began to show signs of acute dysentery within 7 days of the vaccine trial starting. The clinical response of pigs in this vaccination trial was as follows:

| | Unvaccinated | Vaccinated |
|---|---|---|
| Diarrohoea | 110/110 | 2/110 |
| Dysentery | *96/110 | 1/110 |
| Deaths | * 6/110 | 1/110⁺ |

| | | |
|---|---|---|
| * All pigs showing evidence of acute dysentery were treated with 10mg/kg Tiamulin hydrochloride once swine dysentery was detected and confirmed to reduce the deaths that would have inevitably occurred without treatment. | | |
| + One pig in the vaccinated group showed evidence of acute dysentery only 3 days after vaccination and this pig died within a few hours of the onset of the disease. It was considered that 3 days post vaccination was insufficent time to allow for the development of an adequate immune response. | | |

Serological responses of vaccinated and unvaccinated pigs revealed marked differences in the IgG titres to T.hyodysenteriae in infected and uninfected pigs In all vaccinated pigs the serological titre (IgG) to T.hyodysenteriae was greater than 1.0 OD unit at a 1/800 dilution of serum. These vaccination trials demonstrate in pigs vaccinated with oxygen-treated, non-viable strains of T.hyodysenteriae (70A) that an antibody titre (in excess of 1.0 at 1/800) can be obtained that will protect against challenge with a virulent strain of T.hyodysenteriae.
D. Comparison of Immunogenicity.
A comparison was made between adjuvanted live T.hyodysenteriae 70A, and adjuvanted oxygen-treated, non-viable T.hyodysenteriae 70A for ability to stimulate IgG antibodies to T.hyodysenteriae in pigs. Two doses of each vaccine, comprising 1.5 x 10⁹ T.hyodysenteriae in adjuvant were injected intramuscularly, 10 days apart, into 2 groups of 4 pigs. A third group of 4 pigs was held as a control. Sera from all pigs were collected weekly for 3 weeks and the development of the IgG antibody titre to T.hyodysenteriae (as measured by absorbance) was recorded. The results are shown in Fig.6. It can be seen from Fig.6 that there is no significant difference in the absorbance figures induced by either the live or oxygen-treated vaccine.

## Claims

1. A parenteral vaccine composition for the immunisation of pigs against Treponema hyodysenteriae, characterised in that it comprises an oxygen-treated, non-viable strain of T.hyodysenteriae together with an acceptable carrier therefor which is adapted for parenteral administration.

2. A composition according to claim 1, wherein said strain of T.hyodysenteriae is a virulent strain.

3. A composition according to claim 1, wherein said strain of T.hyodysenteriae is an attenuated strain.

4. A composition according to claim 1 or claim 2, wherein said T.hyodysenteriae have been held in oxygen saturated solution to render them non-viable.

5. A composition according to any one of claims 1 to 4, wherein said strain of T.hyodysenteriae is a strain selected from ATCC 31212, ATCC 31287, ATCC 27164 and other strains of T.hyodysenteriae having major abundant proteins of 36 and 37 kDa or 36, 37 and 39 kDa as determined by SDS-PAGE gel electrophoresis of a whole cell lysate.

6. A composition according to any one of claims 1 to 5 further comprising an adjuvant.

7. A composition according to claim 6 wherein said adjuvant is Freund's incomplete adjuvant.

8. A composition as claimed in any one of claims 1 to 7, for use in immunization of pigs against T.hyodysenteriae by parenteral administration.

9. A composition as claimed in claim 8 for use in immunization of pigs against T.hyodysenteriae by intramuscular administration.

10. A method for preparing a vaccine composition for the immunisation of pigs against Treponema hyodysenteriae, comprising the step of exposing cells of Treponema hyodysenteriae to oxygen-treatment, whereby to render them non-viable.

## Patentansprüche

1. Parenterale Vakzinzusammensetzung zur Immunisierung von Schweinen gegen Treponema hyodysenteriae, dadurch gekennzeichnet, daß sie einen Sauerstoff-behandelten, lebensunfähigen T. hyodysenteriae-Stamm zusammen mit einem dafür geeigneten Träger, der an eine parenterale Verabreichung angepaßt ist, umfaßt.

2. Zusammensetzung gemäß Anspruch 1, wobei der T. hyodysenteriae-Stamm ein virulenter Stamm ist.

3. Zusammensetzung gemäß Anspruch 1, wobei der T. hyodysenteriae-Stamm ein attenuierter Stamm ist.

4. Zusammensetzung gemäß Anspruch 1 oder Anspruch 2, wobei T. hyodysenteriae in einer Sauerstoff-gesättigten Lösung gehalten werden, um sie lebensunfähig zu machen.

5. Zusammensetzung gemäß einem der Ansprüche 1 bis 4, wobei der T. hyodysenteriae-Stamm ein Stamm ist, der ausgewählt ist unter ATCC 31212, ATCC 31287, ATCC 27164 und anderen T. hyodysenteriae-Stämmen, die hauptsächlich vorherrschende Proteine mit 36 und 37 kDa oder 36, 37 und 39 kDa aufweisen, was durch SDS-PAGE-Gelelektrophorese eines Gesamtzellysates bestimmt wird.

6. Zusammensetzung gemäß einem der Ansprüche 1 bis 5, die außerdem ein Adjuvans umfaßt.

7. Zusammensetzung gemäß Anspruch 6, wobei das Adjuvans inkomplettes Freund'sches Adjuvans ist.

8. Zusammensetzung gemäß einem der Ansprüche 1 bis 7 zur Verwendung bei der Immunisierung von Schweinen gegen T. hyodysenteriae durch parenterale Verabreichung.

9. Zusammensetzung gemäß Anspruch 8 zur Verwendung bei der Immunisierung von Schweinen gegen T. hyodysenteriae durch intramuskuläre Verabreichung.

10. Verfahren zur Herstellung einer Vakzinzusammensetzung zur Immunisierung von Schweinen gegen T. hyodysenteriae, wobei man in einer Stufe davon T. hyodysenteriae-Zellen einer Sauerstoffbehandlung unterzieht, um diese lebensunfähig zu machen.

## Revendications

1. Composition de vaccin parentéral pour l'immunisation des porcs contre Treponema hyodysenteriae, caractérisée en ce qu'elle comprend une souche traitée à l'oxygène, non viable, de T. hyodysenteriae, ainsi qu'un véhicule acceptable pour celle-ci, adapté à l'administration parentérale.

2. Composition suivant la revendication 1, caractérisée en ce que la souche de T. hyodysenteriae est une souche virulente.

3. Composition suivant la revendication 1, caractérisée en ce que la souche de T. hyodysenteriae est une souche atténuée.

4. Composition suivant la revendication 1 ou la revendication 2, caractérisée en ce que T. hyodysenteriae a été maintenu en solution saturée d'oxygène afin de le rendre non viable.

5. Composition suivant l'une quelconque des revendications 1 à 4, caractérisée en ce que la souche de T. hyodysenteriae est une souche choisie parmi ATCC 31212, ATCC 31287, ATCC 27164 et d'autres souches de T. hyodysenteriae possédant des protéines abondantes majeures de 36 et de 37 kDa ou de 36, 37 et 39 kDa, comme déterminé par électrophorèse en gel de SDS-PAGE d'un lysat de cellules entières.

6. Composition suivant l'une quelconque des revendications 1 à 5, caractérisée en ce qu'elle comprend également un adjuvant.

7. Composition suivant la revendication 6, caractérisée en ce que l'adjuvant est l'adjuvant incomplet de Freund.

8. Composition suivant l'une quelconque des revendications 1 à 7, à utiliser pour l'immunisation de porcs contre T. hyodysenteriae par administration parentérale.

9. Composition suivant la revendication 8, à utiliser pour l'immunisation de porcs contre T. hyodysenteriae par administration intramusculaire.

10. Procédé de préparation d'une composition de vaccin pour l'immunisation de porcs contre Treponema hyodysenteriae, caractérisé en ce qu'il comprend l'étape consistant à exposer des cellules de Treponema hyodysenteriae à un traitement par l'oxygène de manière à les rendre non viables.
